# EUROPEAN PATENT APPLICATION

(11) **EP 1 903 053 A1**
(43) Date of publication of application: **26.03.2008**
(21) Application number: 07018636.6
(22) Date of filing: 21.09.2007
(51) Int. Cl.: C07K 5/10, A61K 38/04, A61Q 19/08, A61K 8/64, A61P 17/00

(54) **Elastin production-enhancing agents**

(30) Priority: 22.09.2006 JP 2006257445
(71) Applicant: Rohto Pharmaceutical Co., Ltd., Osaka-shi, Osaka 544-8666 (JP)
(72) Inventor: Takiguchi, Kumiko, Osaka-shi Osaka 544-8666 (JP); Honma, Yoichi, Osaka-shi Osaka 544-8666 (JP); Kikuchi, Kazuaki, Osaka-shi Osaka 544-8666 (JP); Tsunetsugu, Shuichi, Osaka-shi Osaka 544-8666 (JP)
(74) Representative: Vossius & Partner

(57) **Abstract**

An objective of the present invention is to provide novel useful elastin production-enhancing agents that have a remarkable ability to enhance elastin production. The present invention provides elastin production-enhancing agents that include at least one component selected from the group consisting of the peptide Leu-Glu-His-Ala (formula I), derivatives of the peptide, and salts thereof. The present invention also provides elastin production-enhancing agents that include at least one component selected from the group consisting of peptides which include a conservative substitution, deletion, and/or addition of one or more amino acids in the amino acid sequence of formula I that retain an ability to enhance elastin production in cells; derivatives of such peptides; and salts thereof.

## Description

### FIELD OF THE INVENTION

The present invention relates to novel elastin production-enhancing agents and novel applications associated therewith.

### BACKGROUND OF THE INVENTION

Animal connective tissues are known to contain collagen, hyaluronic acid, elastin, chondroitin sulfate, heparan sulfate, dermatan sulfate, laminin, and the like as major components. As discussed in detail below, elastin in particular plays many important roles in connective tissues.

Elastin is composed of yellow random coil-type fibrous proteins, and is known to have strong elasticity like rubber. In fact, it can be stretched to nearly twice its original length, and still recover upon release. Elastin bridges collagens in connective tissues to support them like a spring, and provides driving forces for expansion and contraction. Thus, elastin plays an important role in maintaining the elasticity and firmness of skin. In the living body, elastin is widely distributed in organs that require flexibility, such as the dermis of skin, ligaments, tendons, and vascular walls, and plays a crucial role in providing elasticity to such organs.

However, the quantity of elastin in the living body is known to gradually decrease due to increasing age, ultraviolet rays, active oxygen, stress, and the like. This decrease contributes to aging by gradually reducing the naturally required elasticity and forming wrinkles or pouches in the skin or such. Furthermore, it has also been known that shortage of normal elastin may cause vascular diseases, such as arteriosclerosis.

Several elastin production-enhancing agents have been proposed to improve conditions associated with reduced elastin. For example, extract of *Polygonatum* plants (Japanese Patent Application Kohyo Publication No. (JP-A) 2001-513509), and extract of *Hippophae* plants, which belong to Elaeagnaceae (Japanese Patent Application Kokai Publication No. (JP-A) 2005-22993 (unexamined, published Japanese patent application)), have been proposed as elastin production-enhancing agents.

### SUMMARY OF THE INVENTION

Due to the importance of elastin in the living body as described above, there is a need in the art to further develop novel useful elastin production-enhancing agents having an ability to markedly enhance elastin production. In view of the conventional problems described above, the present invention aims to provide novel, useful elastin production-enhancing agents capable of markedly enhancing elastin production.

The present inventors conducted dedicated studies to achieve the objective described above. As a result, they discovered that peptides having a specific amino acid sequence had a significantly high ability to enhance elastin production, and thus completed the present invention.

Accordingly, it is an object of the present invention to provide:
(1) an elastin production-enhancing agent comprising at least one component selected from the group consisting of the peptide Leu-Glu-His-Ala (formula I), a derivative of the peptide, and a salt thereof;
(2) an elastin production-enhancing agent, which comprises at least one component selected from the group consisting of:
   a peptide which comprises a conservative substitution, deletion, and/or addition of one or more amino acids in the amino acid sequence of Leu-Glu-His-Ala (formula I) and has an ability to enhance elastin production in a cell;
   a derivative of the peptide; and
   a salt thereof;
(3) the agent of (2), wherein the peptide is three to ten amino acids in length;
(4) the agent of any one of (1) to (3), which further comprises an elastase inhibitor;
(5) a method for enhancing elastin production in a subject, comprising the step of administering to the subject at least one component selected from the group consisting of the peptide Leu-Glu-His-Ala (formula I), a derivative of the peptide, and a salt thereof;
(6) a method for enhancing elastin production in a subject, comprising the step of administering to the subject at least one component selected from the group consisting of:
   a peptide which comprises a conservative substitution, deletion, and/or addition of one or more amino acids in the amino acid sequence of Leu-Glu-His-Ala (formula I) and has an ability to enhance elastin production in a cell;
   a derivative of the peptide; and
   a salt thereof;
(7) the method of (6), wherein the peptide is three to ten amino acids in length;
(8) the method of any one of (5) to (7), which further comprises the step of administering an elastase inhibitor to the subject;
(9) use of at least one component selected from the group consisting of the peptide Leu-Glu-His-Ala (formula I), a derivative of the peptide, and a salt thereof, in the preparation of an elastin production-enhancing agent;
(10) use of at least one component selected from the group consisting of:
   a peptide which comprises a conservative substitution, deletion, and/or addition of one or more amino acids in the amino acid sequence of Leu-Glu-His-Ala (formula I) and has an ability to enhance elastin production in a cell;
   a derivative of the peptide; and
   a salt thereof
   in the preparation of an elastin production-enhancing agent;
(11) the use of (10), wherein the peptide is three to ten amino acids in length; and
(12) the use of any one of (9) to (11), wherein the elastin production-enhancing agent further comprises an elastase inhibitor.

These and other objects and features of the invention will become more fully apparent when the following detailed description is read in conjunction with the accompanying figures and examples. However, it is to be understood that both the foregoing summary of the invention and the following detailed description are of a preferred embodiment, and not restrictive of the invention or other alternate embodiments of the invention. In particular, while the invention is described herein with reference to a number of specific embodiments, it will be appreciated that the description is illustrative of the invention and is not constructed as limiting of the invention.
Various modifications and applications may occur to those who are skilled in the art, without departing from the spirit and the scope of the invention, as described by the appended claims.

### DETAILED DESCRIPTION OF THE INVENTION

The present invention is described in detail below. Although any methods and materials similar or equivalent to those described herein can be used in the practice or testing of embodiments of the present invention, the preferred methods, devices, and materials are now described. However, before the present materials and methods are described, it is to be understood that this invention is not limited to the particular molecules, compositions, methodologies or protocols herein described, as these may vary in accordance with routine experimentation and optimization. It is also to be understood that the terminology used in the description is for the purpose of describing the particular versions or embodiments only, and is not intended to limit the scope of the present invention which will be limited only by the appended claims.

Unless otherwise defined, all technical and scientific terms used herein have the same meaning as commonly understood by one of ordinary skill in the art to which this invention belongs. However, in case of conflict, the present specification, including definitions, will control. Accordingly, in the context of the present invention, the following definitions apply:

As used herein and in the appended claims, the singular forms "a", "an" and "the" include plural reference unless the context clearly dictates otherwise. Thus, for example, reference to a "cell" is a reference to one or more cells and equivalents thereof known to those skilled in the art, and so forth.

The present invention is directed to elastin production-enhancing agents that include at least one component selected from the group consisting of the peptide Leu-Glu-His-Ala (formula I), derivatives of the peptide, and salts thereof. The present invention is also directed to the use of at least one component selected from the group consisting of the peptide, derivatives of the peptides, and salts thereof in the preparation of elastin production-enhancing agents.

Alternatively, the present invention is directed to elastin production-enhancing agents that include at least one component selected from the group consisting of peptides which have a conservative substitution, deletion, and/or addition of one or more amino acids in the amino acid sequence of Leu-Glu-His-Ala (formula I) yet retain the ability to enhance elastin production in cells associated with the original sequence of Formula I; derivatives of such peptides; and salts thereof. The present invention also directed to the use of at least one component selected from the group consisting of these peptides, derivatives of such peptides, and salts thereof in the preparation of elastin production-enhancing agents.

Furthermore, the present invention is directed to methods for enhancing elastin production in a subject, including the step of administering to the subject at least one component selected from the group consisting of the peptide Leu-Glu-His-Ala (formula I), derivatives of the peptide, and salts thereof. In addition, the present invention is directed to methods for enhancing elastin production in a subject, including the step of administering to the subject at least one component selected from the group consisting of peptides which include a conservative substitution, deletion, and/or addition of one or more amino acids in the amino acid sequence of Leu-Glu-His-Ala (formula I) and have an ability to enhance elastin production in a cell; derivatives of the peptides; and salts thereof.

The "at least one component selected from the group consisting of the peptide Leu-Glu-His-Ala (formula I), derivatives of the peptide, and salts thereof" and the "at least one component selected from the group consisting of peptides which comprise a conservative substitution, deletion, and/or addition of one or more amino acids in the amino acid sequence of Leu-Glu-His-Ala (formula I) and have an ability to enhance elastin production in cells; derivatives of the peptides; and salts thereof" are all collectively referred to hereinafter as "LEHA peptides". The use of LEHA peptides in the context of the present invention is described in detail below.

Herein, "derivatives of the peptides" include, but are not limited to, for example, the peptides modified by acetylation, palmitoylation, myristylation, amidation, acrylation, dansylation, biotinylation, phosphorylation, succinylation, anilidation, benzyloxycarbonylation, formylation, nitration, sulfonation, aldehydation, cyclization, glycosylation, monomethylation, dimethylation, trimethylation, guanidylation, amidination, maleylation, trifluoroacetylation, carbamylation, trinitrophenylation, nitrotroponylation, and acetoacetylation. Among these, palmitoylation is preferred, because it is expected to enhance the permeability to cells. Acetylation at N-terminus, and amidation and methylation at C-terminus are also preferred, because they are expected to enhance the stability of the peptide in the living body through conferring resistance against exopeptidases, which degrade peptides from their ends.

Herein, salts of the peptides or their derivatives include any pharmaceutically acceptable salts (including inorganic and organic salts) of the peptides or their derivatives. Such salts include, but are not limited to, for example, sodium salts, potassium salts, calcium salts, magnesium salts, ammonium salts, hydrochlorides, sulfates, nitrates, organic salts (acetates, citrates, maleates, malates, oxalates, lactates, succinates, fumarates, propionates, formates, benzoates, picrates, benzenesulfonates, and so on) of the peptides or their derivatives. Preferred salts include ammonium salts, hydrochlorides, sulfates, and acetates. More preferred salts include ammonium salts and acetates.

Herein, the term "conservative substitution of an amino acid" refers to substitutions selected from among amino acids in each group listed in Table 1 below.

**Table 1**

| | |
|---|---|
| Acidic amino acids | Aspartic acid (D), Glutamic acid (E) |
| Basic amino acids | Arginine (R), Lysine (K), Histidine (H) |
| Hydrophilic amino acids | Serine (S), Threonine (T), Asparagine (N), Glutamine (Q) |
| Hydrophobic amino acids | Tryptophan (W), Phenylalanine (F), Valine (V), Leucine (L), Isoleucine (I), Methionine (M), Proline (P), Alanine (A) |
| Aromatic amino acids | Tyrosine (Y), Tryptophan (W), Phenylalanine (F) |
| Hydroxy amino acids | Serine (S), Threonine (T) |
| Sulfur-containing amino acids | Cysteine (C), Cystine, Methionine (M) |
| Small amino acids | Glycine (G), Alanine (A), Serine (S), Methionine (M), Threonine (T) |

Among these, preferred conservative amino acid substitutions include substitution between aspartic acid (D) and glutamic acid (E); substitution among arginine (R), lysine (K), and histidine (H); substitution between tryptophan (W) and phenylalanine (F); substitution between phenylalanine (F) and valine (V); substitution among leucine (L), isoleucine (I), and alanine (A); and substitution between glycine (G) and alanine (A).

The conservative substitution of one or more amino acids preferably refers to conservative substitution of one or several amino acids, more preferably 1 to 3 amino acids, even more preferably 1 or 2 amino acids, and still more preferably conservative substitution of one amino acid.

The deletion of one or more amino acids preferably refers to deletion of one amino acid.

The addition of one or more amino acids preferably refers to addition of 1 to 6 amino acids, more preferably 1 to 4 amino acids, even more preferably 1 to 3 amino acids, still more preferably 1 or 2 amino acids, and yet more preferably addition of one amino acid.

Peptides having a conservative substitution, deletion, and/or addition of one or more amino acids in Leu-Glu-His-Ala (the peptide sequence of which is referred to hereinafter as LEHA in one-letter code) include, but are not limited to, for example, peptides containing a conservative substitution of one or more amino acids in the LEHA sequence (for example, IEHA, LDHA, LDKA, LEKA, LEHW, LEHF, LEHV, LEHL, LEHI, LEHM, LEHG, LEHS, and LEHT), peptides containing a deletion of one or more amino acids in the LEHA sequence (for example, LEH, EHA, LHA, and LEA), and peptides containing an addition of one or more amino acids in the LEHA sequence (for example, LEHAW, LEHAF, LEHAV, LEHAL, LEHAI, LEHAM, LEHAG, LEHAS, LEHAT, ALEHA, GLEHA, SLEHA, MLEHA, TLEHA, FLEHA, SLEHAT, GLEHAL, DLEHAL, QLEHAK, SLEHAD, QLEHAR, EFLEHA, LEHAVV, DPELEHA, HLEHAAS, and LEHASVD). Among these, preferred peptides include IEHA, LDHA, LDKA, LEKA, LEH, LEHAF, FLEHA, SLEHAT, GLEHAL, DLEHAL, QLEHAK, SLEHAD, QLEHAR, EFLEHA, LEHAVV, DPELEHA, HLEHAAS, and LEHASVD; and more preferred peptides are LEKA, LDHA, LEH, and LEHAF.

Furthermore, peptides containing conservative substitution and deletion of amino acids in the LEHA peptide are not specifically limited, but include, for example, IEH, LDH, LDK, and LEK. In addition, peptides containing deletion and addition of amino acids in the LEHA peptide are not specifically limited, but include, for example, ALEH, GLEH, SLEH, MLEH, and TLEH. Moreover, peptides containing conservative substitution and addition of amino acids in the LEHA peptide are not specifically limited, but preferred examples include IEHAF, LDHAF, LDKAF, and LEKAF.

Herein, the term "having an ability to enhance elastin production in cells" means that the quantity of elastin produced in cells increases when a test substance is allowed to act on the cells, as compared to when the test substance does not act on the cells. The cells in the term mean fibroblasts in specific embodiments, and mean skin fibroblasts in more specific embodiments.

Herein, the terms "subject" and "patient" are used interchangeably herein to refer to the person or animal being treated. In a preferred embodiment, the subject is a mammal, preferably a human.

The peptides used in the present invention can be prepared by methods known in the art. For example, the peptides of the present invention may be synthesized using conventional chemical synthesis methods (for example, solid phase methods (for example, Fmoc method) and liquid phase methods), or prepared using recombinant techniques. Amino acids constituting the peptides of the present invention may take either the L- or D- forms; however, L-forms are preferred.

Alternatively, the peptides of the present invention can be prepared by excising peptides having an amino acid sequence of interest from proteins containing the amino acid sequence using known methods, such as protease treatment. Proteins containing the LEHA sequence include, for example, the proteins listed in Table 2 below.

**Table 2**

| Origin organism | Protein name | Portion containing LEHA sequence | Sequence |
|---|---|---|---|
| Carrot (Daucus carota) | β -fructofuranosidase (EC3.2.1.26) isozyme I class 3 precursor, soluble | Residues 12-15 | LPSRDLEHASSYTP |
| Tomato (Lycopersicon esculentum) | 3β -hydroxylase | Residues 134-137 | IFGSPLEHARQLWP |
| Potato (Solanum tuberosum) | Chaperonin-60 beta subunit precursor | Residues 560-563 | VVRCCLEHAASVAK |
| Rice (Oryza sativa) | Putative chaperonin 60 beta | Residues 562-565 | VVRCCLEHAASVAK |
| Soybean (Glycine max) | glycinin G3 precursor | Residues 228-231 | FAPEFLEHAFVVDR |
| Kidney bean (Phaseolus vulgaris) | Serine threonine kinase homolog COK-4 | Residues 334-337 | EVEVQLEHALSMQE |
| Cassava (Manihot esculenta) | Flavonol 3-O-glucosyltransferase 7 (EC 2.4.1.91) (UDP-glucose flavonoid 3-O-glucosyltransferase 7) (Fragment) | Residues 168-171 | PQVEILEHAALGVF |
| Peach (Prunus persica) | MADS6 | Residues 93-96 | TGSWTLEHAKLKAR |
| Strawberry (Fragaria x ananassa) | UDP-glucose glucosyltransferase | Residues 304-307 | EIANALEHAGHRFL |
| Purple sea urchin (Strongylocentrotus purpuratus) | Hyperpolarization-activated (1h) channel | Residues 406-409 | VAINGLEHAHWWEQ |
| Arctic lamprey (Lethenteron japonicum) | Homeoprotein LjEMX | Residues 193-196 | SQLLRLEHAFEKNH |
| Mussel (Mytilus galloprovincialis) | Paramyosin | Residues 618-621 | DARSLLEHAERARK |

Considering sequence specificity of proteases and such, those skilled in the art can appropriately select proteases suitable to excise peptides composed of the amino acid sequence of interest from proteins that contain such an amino acid sequence. For example, thermolysin (derived from *Bacillus thermoproteolyticus)* and chymotrypsin (derived from bovine pancreas) can be used in combination to excise the LEHA sequence from the carrot-derived protein listed above. In addition, for example, protease M "Amano" G (derived from Aspergillus oryzae, Amano Enzyme Inc.) and the above-mentioned thermolysin can be used in combination to excise the LEHA sequence from the potato-derived protein listed above. In addition, for example, the above-mentioned protease M "Amano" G and the above-mentioned thermolysin can be used in combination to excise the LEHA sequence from the rice-derived protein listed above. In addition, for example, the above-mentioned thermolysin can be used to excise the LEHA sequence from the soybean-derived protein listed above. In addition, for example, the above-mentioned chymotrypsin and the above-mentioned thermolysin can be used in combination to excise the LEHA sequence from the kidney bean-derived protein listed above. In addition, for example, the above-mentioned chymotrypsin and the above-mentioned thermolysin can be used in combination to excise the LEHA sequence from the cassava-derived protein listed above. In addition, for example, trypsin (derived from porcine pancreas) and the above-mentioned thermo lysin can be used to excise the LEHA sequence from the arctic lamprey-derived protein listed above. Combinations of proteases and proteins containing an amino acid sequence of interest are not limited to the combinations indicated above. Preferred combinations include the combination of the soybean protein and thermolysin.

Reaction conditions used to hydrolyze proteins by proteases are not particularly limited, and may be appropriately selected by those skilled in the art using common technical knowledge and routine skill. For example, when commercially-available proteases are used, a reaction condition can be selected according to the manufacturer's published instructions. The reaction temperature can be typically 30 to 80°C, preferably 40 to 70°C, and more preferably 50 to 60°C. The reaction time can be typically 2 to 30 hours, preferably 3 to 24 hours, more preferably 10 to 20 hours, and even more preferably 12 to 18 hours. The reaction pH is preferably near the optimal pH for a protease used. Methods for terminating the reaction are not particularly limited, and include conventional methods known in the art. Such methods include, but are not limited to, for example, heat treatment such as at 85°C for 15 minutes and at 100°C for 5 minutes.

After hydrolysis using proteases, peptides of interest can be obtained through purification using conventional techniques known to the skilled artisan. Such known methods may utilize, for example, a strong acidic ion exchange resin and octadecylsilica (ODS) resin. For example, a peptide of interest can be purified by allowing an aqueous solution of the peptide obtained by protease treatment to adsorb to an ODS resin and eluting the peptide with an arbitrary concentration of an organic solvent (for example, acetonitrile). Alternatively, a peptide of interest can be purified, for example, by allowing an aqueous solution of the peptide obtained by protease treatment to adsorb to a strong acidic ion exchange resin and eluting the peptide with an eluent with a salt concentration of about 0.18 to 0.25 M (for example, sodium chloride and potassium chloride), more preferably a salt concentration of about 0.20 to 0.23 M.

Peptides obtained through hydrolysis of natural proteins using proteases as described above have a cost advantage as compared to those produced by chemical synthesis methods. In addition, peptides obtained through hydrolysis of natural proteins using proteases may be safer for the living body. Thus, peptides obtained as described above can be suitably used in oral medicines, foods, cosmetic products for sensitive skin, animal foods, and the like, which require high safety when applied to the living bodies.

Derivatives of the peptides of the present invention can be prepared by methods known in the art, according to the Fmoc-based solid phase synthesis method (L. A. Carpino, G. Y. Han, J. Am. Chem. Soc., 92, 5748 (1970)) or such.

Salts of the peptides of the present invention can also be prepared by those skilled in the art using any suitable methods known in the art.

In the present invention, the above-described LEHA peptides can be used alone or in any combination of two or more thereof.

The content of LEHA peptides in the elastin production-enhancing agents of the present invention is not particularly limited, as long as they can produce the effect of the present invention. The content is typically about 0.00001 to 100% by weight, preferably about 0.0001 to 70% by weight to the total weight of the elastin production-enhancing agent. In particular, when the elastin production-enhancing agents of the present invention are formulated as an external preparation, the above content may be smaller because they can directly act on the skin. Therefore, in the case of the external preparation, for example, the content of 0.00001 to 5% by weight, preferably 0.0001 to 1 % by weight, and more preferably 0.0001 to 0.1 % by weight, can sufficiently produce the desired effect of the present invention.

In addition to the above-described LEHA peptides, the elastin production-enhancing agents of the present invention may contain one, two or more of additional ingredients, including, but not limited to, skin-whitening ingredients, anti-inflammatory ingredients, anti-microbial ingredients, cell-stimulating ingredients, astringent ingredients, antioxidant ingredients, ingredients for ameliorating acne, ingredients for enhancing synthesis of biological components such as hyaluronic acid, circulation-promoting ingredients, moisturizing ingredients, and anti-aging ingredients. Such additive ingredients can enhance or supplement the activity of the LEHA peptides, or impart other useful activities to the elastin production-enhancing agents of the present invention. Preferably, one, two or more of skin-whitening ingredients, anti-inflammatory ingredients, anti-microbial ingredients, cell-stimulating ingredients, astringent ingredients, antioxidant ingredients, anti-aging ingredients, and moisturizing ingredients are used. These ingredients are not particularly limited, so long as they are suitable for use in the field of pharmaceuticals, quasi drugs, foods, cosmetics, or the like. Accordingly, additive ingredients may be routinely selected and used.

Skin-whitening ingredients suitable for use in the context of the present invention include, but are not limited to, for example, placenta; arbutin; kojic acid; ellagic acid; phytic acid; rucinol; chamomile ET (chamomile extract); and vitamins, such as vitamin A and derivatives thereof, and pantothenic acid and derivatives thereof. Among these, preferred ingredients include pantothenic acid and derivatives thereof, ellagic acid, phytic acid, and vitamin A and derivatives thereof. These skin-whitening ingredients may be used alone or in combination.

Plant components having skin-whitening activity may be used as skin-whitening ingredients. Such plant components include, but are not limited to, those derived from iris, almond, aloe, ginkgo, oolong tea, rose fruit, *Scutellaria* root, *Coptis* rhizome, *Hypericum* plant, *Lamium* plant, seaweed, *Pueraria* root, gardenia, *Sophora* root, chlorella, *Rhus* gall, wheat, rice, rice embryo, oryzanol, rice bran, *Asiasarum* root, *Zanthoxylum* fruit, *Perilla* plant, peony, *Cnidium officinale,* mulberry bark, soybean, natto (fermented soybean), tea, *Angelica* root, *Calendula officinalis,* garlic, hamamelis, safflower, moutan bark, coix seed, *Salvia leucantha, Acacia catechu, Pieris japonica, Pteridium aquilinum, Podocarpus macrophyllus,* nettle tree, *Diospyros kaki, Catalpa* fruit, black bean, gentian, *Scrophularia* root, sarsaparilla, string bean, *Cimicifuga foetida L., Paris polyphylla* rhizome, sage, *Peucedanum root,* radish, azalea, *Lespedeza homoloba, Cuscuta* seed, *Picrasma* wood, parsley, holly, hop, *Lespedeza cyrtobotrya,* clove, licorice, and the like. Preferred ingredients are derived from iris , aloe, ginkgo, oolong tea, rose fruit, *Scutellaria root, Coptis* rhizome, *Hypericum* plant, *Lamium* plant, sea weed, *Pueraria* root, gardenia, *Sophora* root, *Rhus* gall, wheat, rice, rice bran, *Asiasarum* root, *Zanthoxylum* fruit, *Perilla* plant, peony, *Cnidium officinale,* mulberry bark, tea, *Angelica* root, *Calendula officinalis,* hamamelis, safflower, moutanbark, coix seed, *Salvia leucantha, Acacia catechu,* nettle tree, *Diospyros kaki, Catalpa* fruit, black bean, gentian, sarsaparilla, string bean, *Paris polyphylla* rhizome, sage, *Peucedanum* root, radish, azalea, *Lespedeza homoloba, Cuscuta* seed , *Picrasma* wood, parsley, holly, hop, clove, and licorice. More preferred ingredients include those derived from iris, aloe, ginkgo, rose fruit, *Scutellaria* root, *Coptis* rhizome, *Hypericum* plant, gardenia, *Sophora* root, rice, rice bran, *Asiasarum* root, peony, *Cnidium officinale,* mulberry bark, tea, *Angelica* root, *Calendula officinalis,* hamamelis, safflower, moutan bark, *Salvia leucantha, Acacia catechu,* nettle tree, *Diospyros kaki,* sage, radish, azalea, parsley, hop, licorice, and coix seed. When such plant-derived ingredients are used in the elastin production-enhancing agents of the present invention, the form of the plant-derived ingredients are not particularly limited; however, they are typically utilized in the form of plant extract or essential oil.

When the above skin-whitening ingredients are included, their proportion in the elastin production-enhancing agents of the present invention preferably ranges from 0.0003 to 10% by weight, more preferably from 0.01 to 5% by weight.

When the plant-derived components having skin-whitening activity are utilized as skin-whitening ingredients, any single ingredient or combination of two or more ingredients can be used depending on the purpose. When the above-listed plant components are used as skin-whitening ingredients, their proportion in the elastin production-enhancing agents of the present invention typically ranges from 0.00001 to 20% by weight, preferably from 0.0001 to 15% by weight, more preferably from 0.001 to 10% by weight, in terms of extract or essential oil.

Anti-inflammatory ingredients suitable for use in the context of the present invention include, but are not limited to, allantoin, calamine, glycyrrhizinic acid and derivatives thereof, glycyrrhetic acid and derivatives thereof, zinc oxide, guaiazulene, tocopherol acetate, pyridoxine hydrochloride, menthol, camphor, turpentine oil, indomethacin, and salicylic acid and derivatives thereof. Preferred anti-inflammatory ingredients are allantoin, glycyrrhizinic acid and derivatives thereof, glycyrrhetic acid and derivatives thereof, guaiazulene, and menthol.

When the above-listed anti-inflammatory ingredients are included, their proportion in the elastin production-enhancing agents of the present invention preferably ranges from 0.0003 to 10% by weight, more preferably from 0.01 to 5%,by weight.

Anti-microbial ingredients suitable for use in the context of the present invention include, but are not limited to, chlorhexidine, salicylic acid, benzalkonium chloride, acrinol, ethanol, benzethonium chloride, cresol, gluconic acid and derivatives thereof, povidone iodine, potassium iodide, iodine, isopropylmethylphenol, triclocarban, triclosan, photosensitizing dye 101, photosensitizing dye 201, paraben, phenoxyethanol, 1,2-pentanediol, and alkyldiaminoglycine hydrochloride. Preferred anti-microbial ingredients include benzalkonium chloride, benzethonium chloride, gluconic acid and derivatives thereof, isopropylmethylphenol, triclocarban, triclosan, photosensitizing dye 101, photosensitizing dye 201, paraben, phenoxyethanol, 1,2-pentariediol, and alkyldiaminoglycine hydrochloride. More preferred anti-microbial ingredients are benzalkonium chloride, gluconic acid and derivatives thereof, benzethonium chloride, and isopropylmethylphenol.

When the above-listed anti-microbial ingredients are included, their proportion in the elastin production-enhancing agents of the present invention preferably ranges from 0.0003 to 10% by weight, more preferably from 0.01 to 5% by weight.

Cell-stimulating ingredients suitable for use in the context of the present invention include, but are not limited to, amino acids such as γ-aminobutyric acid and ε-aminocaproic acid; vitamins such as retinol, thiamine, riboflavin, pyridoxine hydrochloride, and pantothenic acids; α-hydroxy acids such as glycolic acid and lactic acid; tannin; flavonoid; saponin; allantoin; and photosensitizing dye 301. Preferred cell-stimulating ingredients are amino acids such as γ-aminobutyric acid and ε-aminocaproic acid; and vitamins such as retinol, thiamine, riboflavin, pyridoxine hydrochloride, and pantothenic acids.

When the above-listed cell-stimulating ingredients are included, their proportion in the elastin production-enhancing agents of the present invention preferably ranges from 0.0003 to 10% by weight, more preferably from 0.01 to 5% by weight.

Astringent ingredients suitable for use in the context of the present invention include, but are not limited to, metal salts such as alum, chlorohydroxy aluminum, aluminum chloride, aluminum salt of allantoin, zinc sulfate, and aluminum potassium sulfate; and organic acids such as tannic acid, citric acid, lactic acid, and succinic acid. Preferred astringent ingredients are alum, chlorohydroxy aluminum, aluminum chloride, aluminum salt of allantoin, aluminum potassium sulfate, and tannic acid.

When such astringent ingredients are include, their proportion in the elastin production-enhancing agents of the present invention typically ranges from 0.0003 to 10% by weight, preferably 0.01 to 5% by weight, and more preferably from 0.01 to 3% by weight.

Antioxidant ingredients suitable for use in the context of the present invention include, but are not limited to, butylhydroxyanisol, dibutylhydroxytoluene, sodium bisulfite, erythorbic acid and salts thereof, flavonoid, glutathione, glutathione peroxidase, glutathione-S-transferase, catalase, superoxide dismutase, thioredoxin, taurine, thiotaurine, and hypotaurine. Preferred antioxidant ingredients are thiotaurine, hypotaurine, thioredoxin, and flavonoid.

When such antioxidant ingredients are included, their proportion in the elastin production-enhancing agents of the present invention typically ranges from 0.00001 to 10% by weight, preferably 0.0001 to 5% by weight, and more preferably from 0.001 to 5% by weight.

Anti-aging ingredients suitable for use in the context of the present invention include, but are not limited to, retinoids (retinol, retinoic acid, retinal, and the like), pangamic acid, ursolic acid, turmeric extract, sphingosine derivatives, silicon, silicic acid, N-methyl-L-serine, and mevalonolactone. Preferred anti-aging ingredients are retinoids (retinol, retinoic acid, retinal, and the like).

When the above-listed anti-aging ingredients are included, their proportion in the elastin production-enhancing agents of the present invention preferably ranges from 0.0003 to 10% by weight, more preferably from 0.01 to 5% by weight.

Moisturizing ingredients suitable for use in the context of the present invention include, but are not limited to, amino acids and derivatives thereof, such as alanine, serine, leucine, isoleucine, threonine, glycine, proline, hydroxyproline, glucosamine, and theanine; polyalcohols, such as glycerin, 1,3-butylene glycol, propylene glycol, and polyethylene glycol; sugar alcohols, such as sorbitol; phospholipids, such as lecithin and hydrogenated lecithin; mucopolysaccharides, such as heparin, chondroitin, hyaluronic acid and salts thereof (for example, sodium hyaluronate), acetylated hyaluronic acid and salts thereof (for example, sodium acetylated hyaluronate); and natural moisturizing factors (NMF), such as lactic acid, sodium pyrrolidonecarboxylate, and urea. Preferred moisturizing ingredients are alanine, serine, glycine, proline, hydroxyproline, glucosamine, theanine, collagen, collagen peptides, glycerin, 1,3-butylene glycol, hydrogenated lecithin, propylene glycol, heparin, chondroitin, hyaluronic acid and salts thereof (for example, sodium hyaluronate), acetylated hyaluronic acid and salts thereof (for example, sodium acetylated hyaluronate), lactic acid, and sodium pyrrolidonecarboxylate.

When such moisturizing ingredients are included, the proportion in the elastin production-enhancing agents of the present invention typically ranges from 0.1 to 10% by weight, preferably 0.1 to 5% by weight, more preferably from 0.5 to 5% by weight.

In certain preferred embodiments, the elastin production-enhancing agents of the present invention may contain elastase inhibitors. The elastin production-enhancing agents containing an elastase inhibitor have the effect of suppressing elastin degradation caused by elastase as well as the effect of enhancing elastin production due to the presence of the LEHA peptides. Thus, with these two effects acting synergistically, such combination agents can drastically increase the elastin content in the living body.

Elastase inhibitors suitable for use in the context of the present invention include, but are not limited to, for example, *Hypericum* extract (extract of *Hypericum erectum* Thunb or *Hypericum perforatum* L., which belongs to Guttiferae), chlorella extract, bilberry leaf extract, - algae extract or brown algae extract, green algae extract, iris root extract, hydrolyzed oil meal of *Cucurbita pepo* seed, hydrolyzed potato protein, *Uncaria gambir* extract, *Pisum sativum* extract, *Fucus* extract, comfrey extract, tormentil extract, and *Tilia cordata* extract. The plant extracts listed above can be obtained by common methods in the art using any extraction solvents (for example, water, ethanol, methanol, acetone, 1,3-butylene glycol, propylene glycol, and mixtures thereof). Preferably, *Hypericum* extract is used as a elastase inhibitor.

When such elastase inhibitors are included, their proportion in the elastin production-enhancing agents of the present invention is not particularly limited, but typically ranges from 0.0001 to 20% by weight, preferably from 0.001 to 10% by weight, and more preferably from 0.01 to 5% by weight.

In addition to the specific ingredients described above, other ingredients that are commonly used in the fields of pharmaceuticals, quasidrugs, cosmetics, foods, or the like can be appropriately included in the elastin production-enhancing agents of the present invention, depending on the purpose or dosage form. Ingredients that can be provided in the agents are not particularly limited, but include, for example, amino acids, alcohols, polyalcohols, sugars, gums, polymers such as polysaccharides, surfactants, solubilizing agents, fats and oils, transdermal absorption-accelerating agents, antiseptic agents, anti-microbial agents, disinfectants, pH adjusters, chelating agents, antioxidants, enzymes, binders, disintegrants, lubricants, fluidizers, refrigerants, minerals, cell activators, revitalizer, excipients, thickeners, stabilizers, preservatives, isotonic agents, dispersing agents, adsorbents, disintegration aids, moistening agents, moisture regulators, desiccants, coloring agents, flavoring agents, aromatics, reducing agents, solubilizers, solubilizing aids, foaming agents, viscosity agents, viscosity enhancers, solvents, bases, emulsifiers, plasticizers, buffers, and brightening agents. In particular, when the elastin production-enhancing agents of the present invention are formulated as external preparations, surfactants, solubilizers, or fats and oils can be contained in the agents to improve the sense of use. Such external preparations preferably contain a transdermal absorption-accelerating agent to produce a more superior effect.

Surfactants suitable for use in the context of the present invention include, but are not limited to, various nonionic surfactants, such as polyoxyethylene (hereinafter abbreviated as POE)-branched alkyl ethers such as POE-octyldodecyl alcohol and POE-2-decyltetradecyl alcohol; POE-alkyl ethers such as POE-oleyl alcohol ether and POE-cetyl alcohol ether; sorbitan esters such as sorbitan monooleate, sorbitan monoisostearate, and sorbitan monolaurate; POE-sorbitan esters such as POE-sorbitan monooleate, POE-sorbitan monoisostearate, and POE-sorbitan monolaurate; glycerin fatty acid esters such as glyceryl monooleate, glyceryl monostearate, and glyceryl monomyristate; POE-glycerin fatty acid esters such as POE-glyceryl monooleate, POE-glyceryl monostearate, and POE-glyceryl monomyristate; POE-hydrogenated castor oil fatty acid esters such as POE-dihydrocholesterol ester, POE-hydrogenated castor oil, and POE-hydrogenated castor oil isostearate; POE-alkylaryl ethers such as POE-octylphenyl ether; glyceryl alkyl ethers such as monoisostearyl glyceryl ether and monomyristyl glyceryl ether; POE-glyceryl alkyl ethers such as POE-monostearyl glyceryl ether and POE-monomyristyl glyceryl ether; polyglyceryl fatty acid esters such as diglyceryl monostearate, decaglyceryl decastearate, decaglyceryl decaisostearate, and diglyceryl diisostearate; and naturally occurring surfactants such as lecithin, hydrogenated lecithin, saponin, sodium surfactin, cholesterol, and bile acid. These surfactants may be used alone or in combination of two or more thereof.

When such surfactants are included, their proportion in the elastin production-enhancing agents of the present invention is not particularly limited, so long as they do not interfere with the effect of the present invention. In general, the proportion of surfactants in the elastin production-enhancing agents can be appropriately selected from within the range of 0.01 to 30% by weight. To ensure the stability of active ingredients in the elastin production-enhancing agents, their proportion preferably ranges from 0.1 to 20% by weight, more preferably from 0.5 to 10% by weight.

Solubilizing agents suitable for use in the context of the present invention include, but are not limited to, for example, lower alcohols such as ethanol; polyalcohols such as glycerin and ethylene glycol; hydrogenated soybean phospholipids, polyoxyethylene sorbitan fatty acid esters, polyoxyethylene lanolin alcohol, polyoxyethylene castor oil, polyoxyethylene hydrogenated castor oil, polyoxyethylene sterols, polyoxyethylene alkyl ethers, polyoxyethylene polyoxypropylene alkyl ethers, and polyoxyethylene alkylphenyl ethers. Preferred solubilizing agents are ethanol, glycerin, ethylene glycol, diethylene glycol, dipropylene glycol, hydrogenated soybean phospholipids, polyoxyethylene sorbitan fatty acid esters, polyoxyethylene lanolin alcohol, polyoxyethylene castor oil, polyoxyethylene hydrogenated castor oil, and polyoxyethylene alkyl ethers. More preferred solubilizing agents are ethanol, glycerin, ethylene glycol, diethylene glycol, dipropylene glycol, and hydrogenated soybean phospholipids. These solubilizing agents may be used alone or in combination of two or more thereof.

When such solubilizing agents are included, their proportion in the elastin production-enhancing agents of the present invention is not particularly limited, so long as they do not interfere with the effect of the present invention. In general, the proportion of the solubilizing agents in the elastin production-enhancing agents can be appropriately selected from within the range of 0.01 to 70% by weight. To ensure the stability of active ingredients in the elastin production-enhancing agents, their proportion preferably ranges from 0.1 to 50% by weight, more preferably from 0.1 to 30% by weight.

Fats and oils suitable for use in the context of the present invention include, but are not limited to, for example, synthetic lipids such as medium-chain fatty acid triglycerides; vegetable oils such as soybean oil, rice oil, rapeseed oil, cottonseed oil, sesame oil, safflower oil, castor oil, olive oil, cocoa oil, camellia oil, sunflower oil, palm oil, flax oil, perilla oil, shea oil, sal oil, coconut oil, Japan wax, jojoba oil, grape seed oil, and avocado oil; animal fats and oils, such as mink oil, egg yolk oil, beef fat, milk fat, and lard; waxes, such as beeswax, whale wax, lanolin, carnauba wax, and candelilla wax; hydrocarbons, such as liquid paraffin, squalene, squalane, microcrystalline wax, ceresin wax, paraffin wax, and vaseline; natural or synthetic fatty acids, such as lauric acid, myristic acid, stearic acid, oleic acid, isostearic acid, and behenic acid; natural or synthetic higher alcohols, such as cetanol, stearyl alcohol, hexyldecanol, octyldecanol, and lauryl alcohol; esters and ethers, such as isopropyl myristate, isopropyl palmitate, octyldodecyl myristate, octyldodecyl oleate, and cholesterol oleate; and silicone oil. These fats and oils may be used alone or in combination of two or more thereof.

When these fats and oils are included, their proportion in the elastin production-enhancing agents of the present invention is not particularly limited, so long as they do not interfere with the effect of the present invention. In general, the proportion of the fats and oils in the elastin production-enhancing agents can be appropriately selected from within the range of 0.01 to 70% by weight. To ensure the stability of active ingredients in the elastin production-enhancing agents, their proportion preferably ranges from 0.1 to 60% by weight, more preferably from 0.1 to 50% by weight.

The elastin production-enhancing agents of the present invention may be formulated in dosage forms that are generally used for pharmaceuticals, quasidrugs, cosmetics, foods, and the like, depending on the intended purpose. In general, the dosage forms are solid, semisolid, and liquid formulations. For example, the elastin production-enhancing agents of the present invention can be formulated in known dosage forms such as tablets (including oral rapid disintegrating tablets, chewable tablets, foaming tablets, troches, and jelly drops), pills, granules, fine granules, powders, hard and soft capsules, dry syrups, liquid preparations (including drinkable preparations, suspensions, and syrups), gels, liposome preparations, extracts, tinctures, lemonades, ointments, and jellies. Alternatively, the agents of the present invention can be mixed with other solvents, commonly-used bases, or the like as necessary to be prepared as paste, mousse, gel, liquid, emulsion, cream, sheet (carried by substrates), aerosol, spray or other desired forms.

These dosage forms can be produced by conventional techniques and methods that are well known in the art. In the case of semisolid preparations, for example, the agents of the present invention can be prepared as paste, mousse, gel, liquid, emulsion, cream, sheet (carried by substrates), aerosol, spray, or other desired forms by combining the LEHA peptides with optional ingredients described above as necessary, and further with other solvents, commonly-used bases, or such, if required.

The elastin production-enhancing agents of the present invention may be prepared as, for example, pharmaceuticals, quasidrugs, foods (including confectionery, health foods, nutraceuticals (including balanced nutritional foods and supplements), foods with nutrient function claims, and foods for specified health uses), and such; however, they can be prepared as any products without limitation as long as they have the effect of the present invention. The agents of the present invention can also be prepared as cosmetics, including makeup cosmetics such as foundations, lipsticks, mascaras, eyeshadows, eyeliners, eyebrow liners, and nail care products; basic cosmetics such as lotions (skin lotions, milky lotions, and the like), creams, oils, packs, gels (gel-like essence, gel creams, and the like); cleansing products, such as facial washes, cleansing agents, and body washes; and bath agents.

The elastin production-enhancing agents of the present invention can be used for any number of purposes. While the agents can be used as preparations for internal or external uses, they are preferably used as external preparations in order to produce a strong effect directly on the skin. Furthermore, the elastin production-enhancing agents of the present invention can be preferably used as research reagents (for example, reagents for *in vitro* research) and the like.

The elastin production-enhancing agents of the present invention can be used to increase elastin level in the living body by enhancing elastin production in cells, because they have the markedly high ability to enhance elastin production. Thus, the elastin production-enhancing agents of the present invention can be used to increase the strength and elasticity of the dermis, ligaments, tendons, vascular walls, bones, cartilages, and the like by increasing the quantity of elastin in the living body. For example, the agents can be preferably used to treat or prevent various disorders associated with a reduction in elastin (for example, to treat or prevent vascular diseases caused by shortage of normal elastin, such as arteriosclerosis) and to treat or prevent cosmetic problems (for example, to prevent and/or improve skin wrinkles or pouches, or to prevent and/or improve reduced skin elasticity or firmness, resulting from the reduction of elastin due to aging, ultraviolet light, active oxygen, stresses, and the like). The elastin production-enhancing agents of the present invention can also be preferably used as experimental reagents or such to reveal elastin production-related conditions.

### EXAMPLES

Hereinafter, the present invention is described in more detail by reference to specific examples. However, the following materials, methods and examples only illustrate aspects of the invention and in no way are intended to limit the scope of the present invention. As such, methods and materials similar or equivalent to those described herein can be used in the practice or testing of the present invention.

### EXAMPLE 1

### Preparation of LEHA peptide

The LEHA peptide was synthesized by the Fmoc-based solid phase synthesis method using a peptide synthesizer (PSSM8; Shimadzu). Then, unreacted material was removed using preparative HPLC to purify the LEHA peptide.

The purified peptide was subjected to analytical reverse phase high performance liquid chromatography [column: µBondasphere 5 µ C18-100 angstrom (inner diameter: 3.9 mm, length: 150 mm), Waters; mobile phase: solvent A (0.1% trifluoroacetate) and a gradient of solvent B (0.1% trifluoroacetate, 90% acetonitrile) from 12% to 17% (0 to 20 min.); flow rate: 1 ml/min; detection: absorbance at 220 nm]. A single sharp peak was observed at 12.8 minutes. The purity was 99%.

### EXAMPLE 2

### Assay for the enhancement of elastin production in skin fibroblasts

Normal human skin-derived fibroblasts (CRL-1836; ATCC) were cultured in 60-mm dishes. More specifically, the cells were plated in dishes at a density of 2500 cells/cm², and cultured until subconfluent at 37°C under 5% carbon dioxide gas and 95% air for 6 days. 3 ml of Dulbecco's Modified Eagle Medium (DMEM) supplemented with 10%(w/w) fetal bovine serum (FBS) was used in the culture. Then, the culture medium was changed to the same medium without FBS (namely, serum-free medium), and the culture was continued for 1 day. Next, the culture medium was changed to 3 ml of serum-free medium supplemented with 1000 µg/ml of the LEHA peptide prepared in Example 1, and the cells were cultured for 72 hours. Meanwhile, the culture in which the medium was changed to 3 ml of serum-free culture medium without the LEHA peptide was used as a control.

After 72 hours of culture, the cells in the dishes containing the LEHA peptide and the control dishes were observed under a microscope. Cell counts were not significantly different between both dishes, and no morphological abnormality was observed in the cells.

After microscopic observation, total RNAs were extracted from the cells in each culture using a commercially-available RNA extraction kit (QIAGEN; product name: Rneasy Mini). cDNAs were synthesized from the obtained total RNAs by a conventional method.

The expression of the elastin-encoding gene was quantified using a kit that contains a set of primers for the elastin gene sequence (Applied Biosystems; product name: TaqMan^{®} Gene Expression Assays, Assay ID: Hs 00355783_ml). The kit was used according to the protocol attached thereto, and subjected to ABI real-time PCR system (device name: ABI PRISM^{®} 7000 Sequence Detection System (Applied Biosystems)) along with the cDNAs prepared as above to quantify the elastin expression.

Based on the quantitation result, the expression level of the elastin gene in the cells cultured in the presence of the LEHA peptide was calculated by taking the expression level in the control as 100%. The result showed that the expression level of the elastin gene in the human normal skin-derived fibroblasts was drastically increased to 467% when the cells were cultured in the medium containing the LEHA peptide. This experimental result demonstrated that the LEHA peptide could significantly enhance the, production of elastin in cells.

Formulation examples are described below, but the present invention is not limited to the examples.

| Formulation example 1: Milky lotion | |
|---|---|
| [Ingredients] | [Percentage] |
| LEHA peptide | 0.01 |
| squalane | 2.0 |
| liquid paraffin | 5.0 |
| cetanol | 0.5 |
| glyceryl monostearate | 2.0 |
| POE (25) cetyl ether | 2.0 |
| glycerin | 4.0 |
| 1,3-butylene glycol | 6.0 |
| pH adjuster | adequate amount |
| preservative | adequate amount |
| flavor | adequate amount |
| purified water | adequate amount |
| | 100.0% (w/w) |

| Formulation example 2: Cream | |
|---|---|
| [Ingredients] | [Percentage] |
| LEHA peptide . | 0.01 |
| thermolysin digest of soybean protein * | 0.5 |
| vaseline | 1.0 |
| squalane | 5.0 |
| liquid paraffin | 10.0 |
| stearic acid | 1.5 |
| stearyl alcohol | 2.0 |
| glyceryl monostearate | 2.0 |
| POE (20) cetyl ether | 3.0 |
| glycerin | 6.0 |
| 1,3-butylene glycol | 8.0 |
| pH adjuster | adequate amount |
| preservative | adequate amount |
| flavor | adequate amount |
| purified water | adequate amount |
| | 100.0%(w/w) |

| | |
|---|---|
| * It contains substantially all of various peptides, which can be obtained by digesting soybean protein with thermolysin (EC3.4.24.27). Average molecular weight is 1500 (measured by gel permeation chromatography (GPC)). | |

| Formulation example 3: Skin lotion | |
|---|---|
| [Ingredients] | [Percentage] |
| LEHA peptide | 0.005 |
| sodium hyaluronate | 0.1 |
| POE (20) sorbitan monoisostearate ester | 0.3 |
| succinic acid | 0.2 |
| sodium succinate | 0.5 |
| trisodium edetate | 0.05 |
| 1,3-butylene glycol | 6.0 |
| preservative | adequate amount |
| flavor | adequate amount |
| purified water | adequate amount |
| | 100.0%(w/w) |

| Formulation example 4: Milky lotion | |
|---|---|
| [Ingredients] | [Percentage] |
| LEHA peptide | 0.01 |
| hypericum extract (elastase inhibitor) | 1.0 |
| squalane | 2.0 |
| liquid paraffin | 5.0 |
| cetanol | 0.5 |
| glyceryl monostearate | 2.0 |
| POE (25) cetyl ether | 2.0 |
| glycerin | 4.0 |
| 1,3-butylene glycol | 6.0 |
| pH adjuster | adequate amount |
| preservative | adequate amount |
| flavor | adequate amount |
| purified water | adequate amount |
| | 100.0%(w/w) |

| Formulation example 5: Beverage | |
|---|---|
| [Ingredients] | [Percentage] |
| LEHA peptides | 0.01 |
| fish collagen peptide | 0.5 |
| vitamin B2 | 0.005 |
| erythritol | 10.0 |
| acidulant | 1.0 |
| sweetener | 1.0 |
| flavor | 0.01 |
| purified water | remainder |
| | 100.0%(w/w) |

### Industrial Applicability

The present invention provides novel useful elastin production-enhancing agents having a markedly high ability to enhance elastin production. The elastin production-enhancing agents of the present invention can be beneficially used to increase elastin levels in the living body by enhancing elastin production in cells.

All patents and publications mentioned herein are incorporated by reference in their entirety. Nothing herein is to be construed as an admission that the invention is not entitled to antedate such disclosure by virtue of prior invention.

While the invention has been described in detail and with reference to specific embodiments thereof, it is to be understood that the foregoing description is exemplary and explanatory in nature and is intended to illustrate the invention and its preferred embodiments. Through routine experimentation, one skilled in the art will readily recognize that various changes and modifications can be made therein without departing from the spirit and scope of the invention. Thus, the invention is intended to be defined not by the above description, but by the following claims and their equivalents.

## Claims

1. A composition comprising at least one component selected from the group consisting of the peptide Leu-Glu-His-Ala (formula I), a derivative of the peptide, and a salt thereof for the enhancement of elastin production.

2. A composition, which comprises at least one component selected from the group consisting of:
a peptide which comprises a conservative substitution, deletion, and/or addition of one or more amino acids in the amino acid sequence of Leu-Glu-His-Ala (formula I) and
has an ability to enhance elastin production in a cell;
a derivative of the peptide; and
a salt thereof for the enhancement of elastin production.

3. The composition of claim 2, wherein the peptide is three to ten amino acids in length.

4. The composition of any one of claims 1 to 3, which further comprises an elastase inhibitor.

5. A composition as defined in any one of claims 1 to 4 for the treatment or prevention of a disorder associated with a reduction in elastin.

6. A cosmetic composition as defined in any one of claims 1 to 4 for the prevention or improvement of skin wrinkles, skin pouches, reduced skin elasticity, or reduced skin firmness.

7. Use of at least one component selected from the group consisting of the peptide Leu-Glu-His-Ala (formula I), a derivative of the peptide, and a salt thereof, for the preparation of a composition for the enhancement of elastin production.

8. Use of at least one component selected from the group consisting of:
a peptide which comprises a conservative substitution, deletion, and/or addition of one
or more amino acids in the amino acid sequence of Leu-Glu-His-Ala (formula I) and
has an ability to enhance elastin production in a cell;
a derivative of the peptide; and
a salt thereof
for the preparation of a composition for the enhancement of elastin production.

9. Use of at least one component selected from the group consisting of the peptide Leu-Glu-His-Ala (formula I), a derivative of the peptide, and a salt thereof, for the preparation of a composition for the treatment or prevention of a disorder associated with a reduction in elastin.

10. Use of at least one component selected from the group consisting of:
a peptide which comprises a conservative substitution, deletion, and/or addition of one
or more amino acids in the amino acid sequence of Leu-Glu-His-Ala (formula I) and
has an ability to enhance elastin production in a cell;
a derivative of the peptide; and
a salt thereof
for the preparation of a composition for the treatment or prevention of a disorder associated with a reduction in elastin.

11. Use of at least one component selected from the group consisting of the peptide Leu-Glu-His-Ala (formula I), a derivative of the peptide, and a salt thereof, for the preparation of a cosmetic composition for the prevention or improvement of skin wrinkles, skin pouches, reduced skin elasticity, or reduced skin firmness.

12. Use of at least one component selected from the group consisting of:
a peptide which comprises a conservative substitution, deletion, and/or addition of one or more amino acids in the amino acid sequence of Leu-Glu-His-Ala (formula I) and
has an ability to enhance elastin production in a cell;
a derivative of the peptide; and
a salt thereof
for the preparation of a cosmetic composition for the prevention or improvement of skin wrinkles, skin pouches, reduced skin elasticity, or reduced skin firmness.

13. The use of any one of claims 8, 10 or 12, wherein the peptide is three to ten amino acids in length.

14. The use of any one of claims 7 to 13, wherein the composition further comprises an elastase inhibitor.


SEQUENCE LISTING

<110>  ROHTO PHARMACEUTICAL CO., LTD.

<120>  ELASTIN PRODUCTION-ENHANCING AGENTS

<130>  34-060-01

<160>  13

<170>  PatentIn version 3.1

<210>  1
<211>  4
<212>  PRT
<213>  Artificial Sequence

<220>
<223>  the peptide usable in the present invention

<400>  1

Leu Glu His Ala
1

<210>  2
<211>  14
<212>  PRT
<213>  Daucus carota

<400>  2

Leu Pro Ser Arg Asp Leu Glu His Ala Ser Ser Tyr Thr Pro
1               5                   10

<210>  3
<211>  14
<212>  PRT
<213>  Lycopersicon esculentum

<400>  3

Ile Phe Gly Ser Pro Leu Glu His Ala Arg Gln Leu Trp Pro
1               5                   10

<210>  4
<211>  14
<212>  PRT
<213>  Solanum tuberosum

<400>  4

Val Val Arg Cys Cys Leu Glu His Ala Ala Ser Val Ala Lys
1               5                   10

<210>  5
<211>  14
<212>  PRT
<213>  Oryza sativa

<400>  5

Val Val Arg Cys Cys Leu Glu His Ala Ala Ser Val Ala Lys
1               5                   10

<210>  6
<211>  14

<212>  PRT
<213>  Glycine max

<400>  6

Phe Ala Pro Glu Phe Leu Glu His Ala Phe Val Val Asp Arg
1               5                   10

<210>  7
<211>  14
<212>  PRT
<213>  Phaseolus vulgaris

<400>  7

Glu Val Glu Val Gln Leu Glu His Ala Leu Ser Met Gln Glu
1               5                   10

<210>  8
<211>  14
<212>  PRT
<213>  Manihot esculenta

<400>  8

Pro Gln Val Glu Ile Leu Glu His Ala Ala Leu Gly Val Phe
1               5                   10

<210>  9
<211>  14
<212>  PRT
<213>  Prunus persica

<400> 9

Thr Gly Ser Trp Thr Leu Glu His Ala Lys Leu Lys Ala Arg
1               5                   10

<210> 10
<211> 14
<212> PRT
<213> Fragaria x ananassa

<400> 10

Glu Ile Ala Asn Ala Leu Glu His Ala Gly His Arg Phe Leu
1               5                   10

<210> 11
<211> 14
<212> PRT
<213> Strongylocentrotus purpuratus

<400> 11

Val Ala Ile Asn Gly Leu Glu His Ala His Trp Trp Glu Gln
1               5                   10

<210> 12
<211> 14
<212> PRT
<213> Lethenteron japonicum

<400> 12

Ser Gln Leu Leu Arg Leu Glu His Ala Phe Glu Lys Asn His

1                    5                        10

<210>  13
<211>  14
<212>  PRT
<213>  Mytilus galloprovincialis

<400>  13

Asp Ala Arg Ser Leu Leu Glu His Ala Glu Arg Ala Arg Lys
1                    5                        10